# EUROPEAN PATENT APPLICATION

(11) **EP 1 493 412 A2**
(43) Date of publication of application: **05.01.2005**
(21) Application number: 04253887.6
(22) Date of filing: 29.06.2004
(51) Int. Cl.: A61F 13/15

(54) **Absorbent article including in situ cover**

(30) Priority: 30.06.2003 US 609736
(71) Applicant: McNeil-PPC, INC., Skillman NJ 08558 (US)
(72) Inventor: Dabi, Shmuel, Higland Park, New Jersey 08904 (US); Disalvo, Anthony L., Bernardsville, New Jersey 07924 (US); Haddack, Teresa H., University Park, Florida 34201 (US); Hull, Raymond J. Jr., Hampton, New Jersey 08827 (US); Luizzi, Joseph M., Newtown, Pennsylvania 18940 (US); Nguyen, Hien Vu, East Windsor, New Jersey 08520 (US); Yang, Ching-Yun M., Princeton Junction, New Jersey 08850 (US)
(74) Representative: Mercer, Christopher Paul

(57) **Abstract**

An absorbent article including an absorbent structure is disclosed. The absorbent structure has an absorbent core and a plurality of discrete entities deposited on the absorbent core, wherein the combination of absorbent core and discrete entities form a body facing surface of the absorbent article.

## Description

### Field of the Invention

The invention relates to disposable absorbent articles for body exudates. In particular, the absorbent articles of the present invention are dispersible in water and may be disposed in a standard toilet system.

### Background of the Invention

Disposable absorbent articles such as, pantiliners, sanitary napkins, interlabial devices, adult incontinence devices, bandages, and diapers are well known in the art. These articles typically have a fluid permeable body-facing side and fluid impermeable garment facing side. Additionally, such articles may include an absorbent core for retaining fluids therebetween.

Typical disposal methods of such articles are generally considered to be unsatisfactory as the article usually ends up being incinerated or in a landfill.

An early method to overcome this problem is disclosed in U.S. Pat. No. 3,683,919 (Ellis). Ellis purports to disclose mechanical method for achieving a flushable sanitary napkin having a topsheet, absorbent core and backsheet. In this method, the topsheet and backsheet are torn apart and separated from the absorbent core prior to flushing. This method is often messy and inconvenient to the user.

Such mechanical methods do not overcome the mess and inconvenience faced by the user who wishes to dispose of the sanitary napkin by flushing down the toilet. Optimally, a more acceptable disposal method involves flushing the article down the toilet without any mechanical alteration by the user to the used sanitary napkin. For this disposal method, the materials used should not only maintain their structural integrity for the napkin's intended use, but also have the ability to disperse into small entities when flushed down a conventional toilet without causing blockage of the plumbing system. The materials also need to be stable and maintain integrity during storage, particularly in humid environments, such as, a bathroom or a warehouse.

Commercial absorbent articles typically include a body facing liquid permeable layer, an absorbent core and garment facing liquid impermeable barrier. Some of the materials used for such components may show the desired flushability but lack stability, often losing strength and shape upon use. Other materials show stability but lack the ability to be safely flushed in normal sewage systems. For example, commercially available water soluble polymers such as polyethylene oxide (PEO), polyvinyl alcohol (PVOH), acrylamide polymers, acrylic acid-based polymers, and cellulose derivatives possess the desired characteristics for flushability but typically are unstable in use and storage. In particular, these polymers may get tacky in a moist environment including when handled by the user. Additionally, the disposable, flushable absorbent product should be relatively responsive to water and agitation so that it is transportable in the sewer system.

Some materials have been proposed in an attempt to improve the flushability of absorbent articles by using specific materials that weaken when exposed to liquid or are biodegradable. Designing an absorbent article having a fluid impervious barrier that weakens or degrades when exposed to liquid is desirable. See, for example, U.S. Pat. Nos. 6,359,063 ('603) and 6,362,277 ('277) (both to Wang).

The '603 and the '277 purport to disclose flushable personal care articles having water-degradable polyolefin-containing film. In '602, film is made from grafting a monomer by free radical initiator onto a film made of a mixture of modified polyolefin and modified poly(ethylene oxide). In '277, poly(ethylene oxide) is unmodified. The components of the film are melt blended and extruded. The films lose at least 10 percent in two or more tensile properties after being immersed in water for 30 seconds.

Fluid impervious tissue or paper may also be used for the barrier. In general, paper is a material in which cellulose natural fibers are accumulated in sheet form and in which the fibers are linked by hydrogen bonds. When immersed in water, the hydrogen bonds are broken and the fiber is readily dispersed into fiber units. An example is toilet paper.

Paper is a type of material that exhibits too little wet strength to be useful in absorbent articles, some which must function in the presence of large amounts of moisture. In general, some paper tends to also be too stiff in their dry state to provide the comfort most consumers expect. To overcome these problems, paper may be coated or further treated to improve its physical properties. Examples of such treatments of cellulosic materials including wood pulp and cellulosic fibers can be found in U.S. Pat. Nos. 3,884,987, 4,057,537, and 5,015,245.

The dispersibility and solubility of water soluble paper differs from that of ordinary paper. When components, such as, modified starches, including carboxymethylcellulose (CMC) and hydroxymethyl cellulose (HMC), polyacrylate, polyvinyl alcohol are added to cellulose fibers, the resulting paper becomes water-soluble as it has a water soluble polymer that partially swells and dissolves.

An example of a disposable absorbent article is disclosed in EP 1166803 (Shimizu). This invention purports to provide a water decomposable absorbent article having a backsheet formed of a fibrous sheet containing water-dispersible fibers and water-insoluble CMC where the CMC has a degree of esterification of between 0.3 and 0.6 and modified such that the hydrogens of at least 95% of carboxylic acids are substituted with metal. The CMC serves as a binder in the fibrous sheet in dry condition. However, the material described by Shimizu is very water soluble and loses integrity immediately upon exposure to body exudates.

Ishikawa et al. in "Functional Paper in Specialty Paper" (Shikisai, Vol. 64, pp. 696-701 1991) discloses water soluble paper in which CMC is compounded with paper in order to increase the characteristic of dispersion in water so that it can be dispersed and dissolved in water rapidly in the short time of 5 to 20 seconds.

In addition, others have sought to apply a polymer film or foam coatings for the covers of absorbent articles. (See U.S. Pat. No. 5,454,801 "Lauritzen".) Such disclosure is directed to solving the problem of providing an outer surface coating that contacts the human body to overcome the uncomfortable and irritating problems associated with apertured polymer films. More particularly, Lauritzen discloses absorbent products and processes for making in situ foamed polymer coatings which give an opaque, soft, dry and clean-appearing water-permeable cover to absorbent products such as sanitary napkins, diapers and the like. Additionally, the coatings disclosed were continuous coatings. Such coatings tend to hold the article together, thereby impeding the breaking apart of the absorbent article. Additionally, Lauritzen does not disclose that the absorbent articles are flushable and/or biodegradable..

What is needed is an absorbent article that is flushable, that retains its integrity during use but will break apart upon exposure to water.

### Summary of the Invention

An absorbent article comprising, consisting of, and/or consisting essentially of an absorbent structure, the absorbent structure comprising an absorbent core and a plurality of discrete entities deposited on the absorbent core, wherein the combination of absorbent core and discrete entities form a body facing surface.

An absorbent article comprising, consisting of, and/or consisting essentially of an absorbent structure, the absorbent structure having an absorbent core and a plurality of discrete entities deposited on the absorbent core, wherein the combination of absorbent core and discrete entities form a body facing surface, and a garment facing layer juxtaposed to the absorbent structure opposite the body facing surface.

### Brief Description of the Drawings

FIG. 1 is a plan view of a pantiliner according to the invention;
FIG. 2 is a cross sectional of the pantiliner taken along line 2-2 of FIG. 1;
FIG. 3 is a cross section of an alternate embodiment of the invention; and
FIG. 4 is a cross section of another alternate embodiment of the invention.

### Detailed Description of the Invention

"Absorbent article" denotes any article that absorbs body exudates or fluids. Included in the phrase are sanitary napkins, pantiliners, incontinence devices, bandages such as surgical bandages and minor wound covering, shoe insoles, breast pads, under arm pad, and the like.

"Agitation" as used herein means to move with irregular, rapid or violent action. In particular, agitation denotes the action of the water during flushing of any typical toilet or urinal. Normally, agitation possesses sufficient energy (of the water movement) to break apart the absorbent article or entities into smaller portions. These smaller portions are easily flushed and pose no threat to conventional water/sewer systems. Not all toilets have equivalent agitation; some newer toilets flush using gravity. Agitation may also include the force of a vortex formed when stirring water in a flask or beaker.

"Biodegradable" as used herein means those materials that are capable of being broken down especially into innocuous products by the action of living things (such as microorganisms). In this disclosure, the term "biodegradable" is used for that type of degradability that is brought about by living organisms, usually microorganisms.

"Breaks apart" as used herein means the article or element exhibits visible changes after being flushed down a standard toilet; the changes may include any visible failures to the integrity of the article or element, such as holes, slits, shreds; breaking apart into smaller sections; dissolving; or a combination thereof.

"Flushable" as used herein means discardable in a toilet, urinal, or other flushing device made for the purpose of receiving urine or other body exudates and transporting it through a public or private sewage or plumbing system.

"Discrete" as used herein means distinct, unconnected, discontinuous entities.

"Water insoluble" as used herein relates to those materials that resist being dissolved or broken apart in water.

All ranges provided herein are intended to expressly include all values between the endpoints of the ranges.

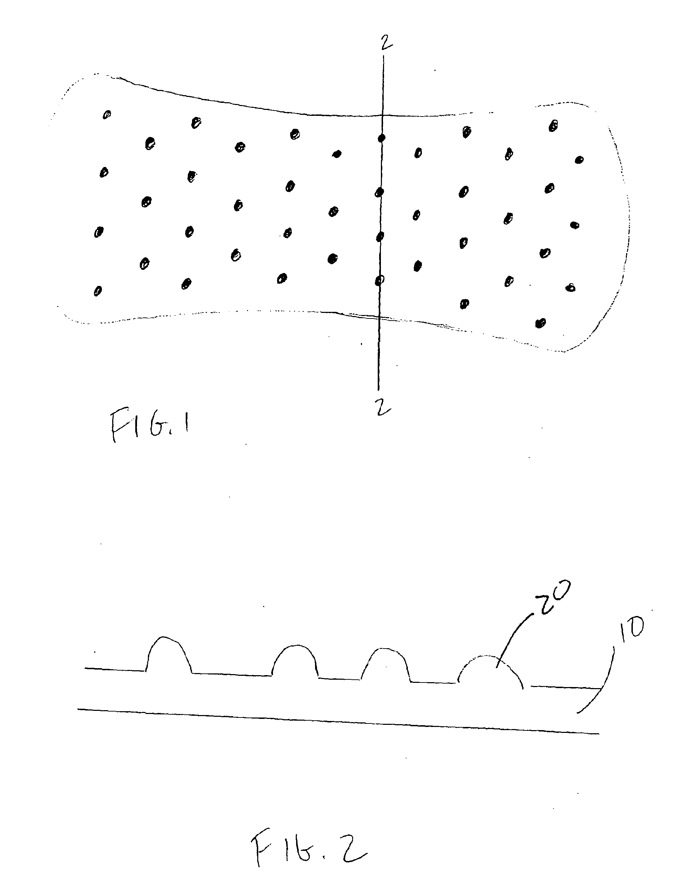

Figures 1-4 show various embodiments of the absorbent article, each embodiment having an absorbent core 10 and discrete entities 20.

### Cover

In this invention, the body facing surface of the absorbent article contains discrete entities to provide dryness, comfort, and product integrity. Such discrete entities do not impede the breaking apart of the absorbent article because they do not hold the absorbent article together.

In one embodiment of the present invention, the coating on the body facing surface provides a structure having both fluid impervious and fluid pervious areas to provide a mechanism to direct fluid flow. In another embodiment of the present invention, the coating on the body facing surface maintains the fluid pervious properties of the absorbent core.

In this invention, the coating on the body facing surface provides discrete entities. The discrete entities may be deposited onto portions of the surface or onto the entire surface. In another embodiment, the garment facing surface may contain discrete entities. In still another embodiment, both the body facing surface and the garment facing surface have discrete entities deposited thereon.

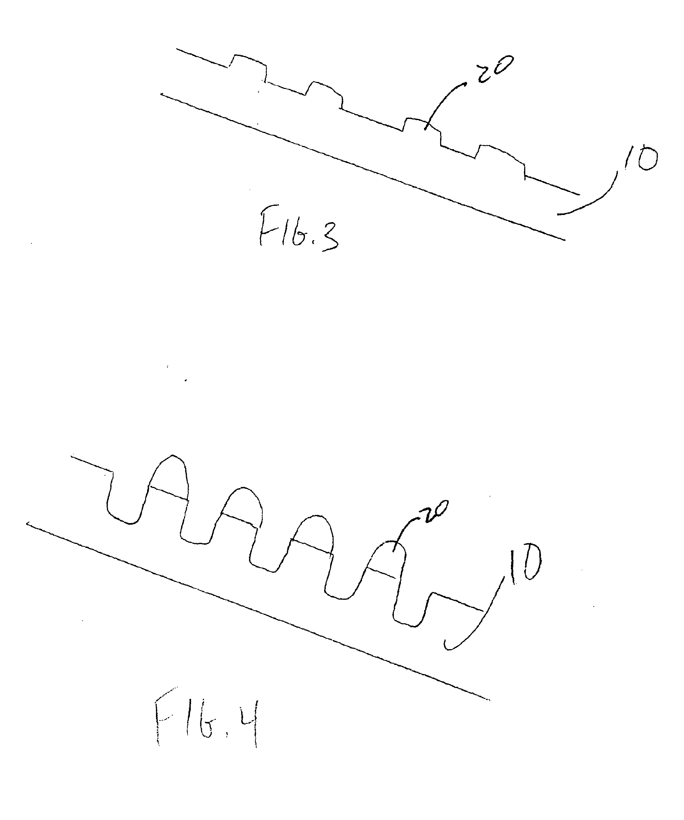

The discrete entities may be any shape or configuration. Examples of shapes include, but are not limited to, circular dots (raised (Figure 2) or flattened (Figure 3)), hexagon, rectangles, hearts, stars, triangles, diamonds, squares, ovals, lines, waves, flowers, petals etc. While not being wished to be bound by any particular theory, it was found that the larger the size of the discrete entity the better the adherence to the substrate by the discrete entity because smaller discrete entities have less penetration into the substrate.

The discrete entities can cover up to 60% of the total surface area of substrate, leaving at least about 40% of the area "open" or uncoated. In one embodiment, the discrete entities are hydrophobic and the resulting open area of the absorbent core is hydrophilic. The open area readily accepts fluid.

In an embodiment where the article is flushable, there is a relationship between the dot-to-dot distance and the fiber length of the absorbent core. The average fiber length should not exceed the dot-to-dot distance to avoid adhering multiple fibers together and forming a network, thus impeding break apart properties.

Suitable coating materials used to produce the discrete entities include, but are not limited to, hot melt coatings; natural rubber; synthetic rubber; polyolefins, such as, polyethylene and polypropylene; ethylene vinyl acetate; and thermoplastic elastomers. Colorants, pigments, or fragrances may be combined with the coating materials.

Suitable hot melt coatings for generating raised entities include HL-7471 W, from H.B. Fuller Co., St. Paul, MN, and REXTAC amorphous polyolefins, available through Huntsman Chemical. Hot melt coatings containing from about 15% to about 100% olefin polymer or a block copolymer, from about 0% to about 60% tackifying resin, and from about 0% to about 50% wax may be useful. In addition, the use of fillers in the coating may also be beneficial to reduce costs, add process benefits, e.g., thixotropy. or to provide masking or whitening benefits. Examples of such fillers include, but are not limited to, fumed silica, such as, Cabosil, from Cabot Corp., calcium carbonate, titanium dioxide, zinc oxide, magnesium oxide, wood flour, or diatomaceous earth.

Additionally, water dispersible sulfopolyester hot melt adhesive (available from National Starch, Bridgewater, NJ ) can be used to form a series of discrete entities on the surface of the absorbent core. The adhesive is deposited on the surface, allowed to solidify to form discrete entities. Eastman AQ water-dispersible polyesters (from Eastman Chemical) may also be used.

Suitable olefin polymers include polymers: a) wherein the olefin polymer is a homopolymer of ethylene, propylene, n-butene, butylene or isobutylene, with a melt flow index from 0.5 to 2500, such as Ateva™ polymers from AT plastics; Escorene® and Vistanex® polymers from Exxon Chemical, Duraflex® polymers from Shell Chemical, Epolene® polymers from Eastman Chemical, and Vestoplast® polymers from Creanova; b) wherein the olefin polymer is a copolymer of ethylene and a comonomer, such as vinyl acetate, acrylic acid, methacrylic acid, ethyl acrylate, methyl acrylate, n-butyl acrylate vinyl silane or maleic anhydride, such as Ateva® polymers from AT plastics, Elvax® polymers from DuPont, Escorene® and Optema® polymers from Exxon Chemical, and Primacor® polymers from Dow Chemical; and c) wherein the olefin polymer is a terpolymer of ethylene and co-monomers, such as vinyl acetate, acrylic acid, methacrylic acid, ethyl acrylate, methyl acrylate, n-butyl acrylate vinyl silane or maleic anhydride, such as Ateva® polymers from AT plastics, Nucrel® polymers from DuPont, and Escor® polymers from Exxon Chemical.

Suitable block copolymers include block copolymers having a linear or a radial structure such that the structure (A--B), where A is consists essentially of a polyvinylarene block, and B consists essentially of poly(monoalkenyl) block, and x denotes the number of polymeric arms, where x is greater than or equal to one are also useful. Block B may be selected from conjugated diene elastomers such as polybutadiene or polyisoprene and hydrogenated elastomers such as ethylene-butylene or ethylene- propylene. Suitable examples of these types of polymers include Kraton® elastomers from Kraton Ploymer Co, Vector® elastomers from Dexco, Solprene® elastomers from Enichem Elastomers and Stereon® from elastomers Firestone Tire & Rubber Co. When the hot melt coatings contain block copolymers, it is preferable for the coating to contain from about 15% to about 50% block copolymer.

Suitable tackifying resins include any compatible resin or mixture thereof selected from the group consisting of a) natural and modified rosins; b) glycerol and pentaerythritol esters of natural and modified rosins; c) polyterpene resins; d) copolymers and terpolymers of natural terpenes; e) phenolic modified terpene resins and the hydrogenated derivatives thereof; f) aliphatic petroleum resins and the hydrogenated derivatives thereof; g) aromatic petroleum resin and the hydrogenated derivatives thereof; and h) aliphatic/aromatic petroleum resins and the hydrogenated derivatives thereof, such as Foral® resin, Staybelite® resin, Poly-pale® resin, Permalyn® resin, Pentalyn® resin, Adtac® resin, Piccopale® resin, Piccotac® resin, Hercotac® resin, Regalrez® resin, and Piccolyte® resin from Hercules, Escorez® resin from Exxon Chemical, Wingtack® resin from Goodyear Tire & Rubber Co., Arkon® resin from Arakawa Chemicals, Zonatac® resin, Zonarez® resin and Zonester® resin from Arizona Chemical and Nevtac® resin from Neville Chemical Company.

Suitable waxes include, but are not limited to, paraffins, Fischer-Tropsch, microcrystalline waxes, and combinations thereof. Suitable microcrystalline waxes include, but are not limited to, BE SQUARE 175 microwax, available from Bareco Division, Petrolite Corporation, and M-5165 from Moore & Munger, Shelton, CT. Suitable polyethylene waxes include, but are not limited to, H-101 from Exxon Chemical, Houston, TX. Suitable Fischer-Tropsch waxes include, but are not limited to, Paraflint Wax from Schumann Sasol, Hamburg, Germany.

Discrete entities can be deposited on the surface of a substrate by any method known in the art, including, but not limited to, gravure printing, screen printing or coating, kiss coating, porous roll printing, flexo printing, reverse roll coating, transfer coating and the like.

### Absorbent Core

The materials selected for the absorbent core of the present invention are dependent upon whether the absorbent article is a flushable absorbent article or a non-flushable absorbent article. Generally, the absorbent core can be a fluffy batt cut from a relatively loose web of non-woven fibers having absorptive properties. The absorbent core may also be a fibrous batt having an integral densified layer. In such a case, if a backsheet is desired, the absorbent core is positioned on the backsheet of the absorbent article so that the densified layer adjoins the backsheet. The densified layer has relatively higher wettability and liquid retentivity than the rest of the aforesaid batt and usually is formed by slightly moistening one surface of the batt and thereafter compressing the moistened surface. The absorbent core may also be formed from multiple layers, each having a different density such that the uppermost layer (closest to the body) is less dense than the outer (closest to the garment).

The absorbent core of the present invention may contain any known absorbent materials including, but not limited to, absorbent fibers, such as, cellulose fibers, including, but not limited to wood pulp, regenerated cellulose fibers, and cotton fibers, rayon fibers and the like; superabsorbent fibers or particles; other naturally occurring absorbent materials, such as sphagnum or peat moss; and other synthetic absorbent materials, such as foams and the like. The absorbent core may also include one or more of the following: thermoplastic binder fibers, latex binder, perfumes, or odor-controlling compounds.

Where the absorbent article of the present invention is a flushable absorbent article, the fibers used are short cut fibers or staple fibers in the range of from about 1 to about 12 mm and are calendared to a density of about 0.2 to about 0.6 g/cc to facilitate breaking apart. In addition, an absorbent core used in a flushable absorbent article may not contain any binders and can be made by a wet-laid process or a compression method. Where the absorbent article of the present invention is not flushable, any fiber length can be used to make up the absorbent core.

While the absorbent core can have any shape or silhouette, it usually has an asymmetric configuration.

In one embodiment, the absorbent core is made of absorbent material that is made from a layer of pulp. In another embodiment, superabsorbent polymer (SAP) is mixed with the pulp to form an absorbent composite. This composite may be condensed to form a dense, thin layer. One example of such a material is NovaThin® brand absorbent core (from Rayonier).

Superabsorbent polymers are made from particles that are capable of absorbing many times, preferably 10, more preferably 15, and still more preferably over 15, their weight in exudate, under a pressure of 0.5 psi. It should be noted that, in the context of the present invention, there is no restriction that the superabsorbent particles, actually be particulate. This expression is intended to cover superabsorbent fibers, and other superabsorbent materials, whatever their form and shape. These superabsorbent particles generally fall into three classes, namely starch graft copolymers, cross-linked carboxymetylcellulose derivates and modified hydrophilic polyacrylates. Examples of such absorbent polymers are hydrolyzed starch-acrylonitrile copolymer graft copolymer, a neutralized starch-acrylic acid graft copolymer, a saponified acrylic acid ester-vinyl acetate copolymer, a hydrolyzed acrylonitrile copolymer or acrylamide copolymer, a modified cross-linked polyvinyl alcohol, a neutralized self-cross-linking polyacrylic acid, a cross-linked polyacrylate salt, carboxylated celluslose, and a neutralized cross-linked isobutylene-malsic anhydride copolymer. In one embodiemnt of the invention, the superabsorbent particle is a cross-linked polyacrylate salt.

The superabsorbent particles are incorporated into the absorbent core in an amount no greater than about 60% on a weight per weight basis. Preferably, they are incorporated in an amount between about 0% and about 25% on a weight per weight basis. More preferably, they are incorporated in an amount between about 15% and about 20% on a weight per weight basis. For example, in the present context, 12% superabsorbent on "a weight per weight basis" is meant to mean 0.12 grams of superabsorbent particles per 1 gram of all components in the absorbent core.

While not wishing to be bound to any particular theory, in one embodiment, it is believed that when using a SAP-containing absorbent core coated with discrete entities, upon exposure to toilet flushing, the expansion of the SAP destroys the integrity of the layer causing the layer to dissipate into smaller portions. These portions are easily flushed through any conventional sewage system.

In another embodiment, the absorption of water and agitation is sufficient to dissipate the absorbent core and discrete entities into smaller portions.

The absorbent core of the present invention may be constructed according to conventional techniques, e.g., by air-laying wood pulp or a mixture of wood pulp fibers and superabsorbent material. All such conventional techniques are within the scope of the present invention. In one embodiment, an absorbent core as described in U.S. Pat. No. 5,866,242 to Tan et *al*., which is herein incorporated by reference in its entirety, can be used.

Additionally, the absorbent material can be an offline-formed, homogeneously mixed, air-laid layer, roll good laminate or any other offline-formed absorbent composite.

The ratio of SAP to wood pulp may be varied over a wide range. If desired, a layer or multilayer of drylaid type material can be used as the absorbent material to form the absorbent core. The absorbent material may be made of a superabsorbent polymer (SAP) of the type used in the art and wood pulp fibers having the desired density.

The absorbent core may also include additional materials, such as, binders, odor control material, wetness indicator material, materials for administering or delivering medicaments, such as, encapsulated medicaments, and materials for maintaining skin moisture, such as encapsulated moisturizers.

The absorbent material may be treated with any known wetting agents or surfactants. For example, polyethylene glycol and propylene glycol from Union Carbide, polyoxyethylene sorbitan fatty acid esters, e.g., TWEEN® 20 from Unqema, sorbitan fatty acid esters, e.g., SPAN® from Uniquma, Plantaren® from Cognis, and Sandopan LS-24 from Clariant.

The absorbent core may be compressed or uncompressed, embossed or unembossed, or calendered. Embossing may be done by any conventional methods known in the art that result in at least one boss and at least one valley disposed on the substrate's surface. While any length of boss to valley can be used, a length of at least about 1 mm is particularly useful.

In one embodiment, embossing and coating can be done using a single station that provides perfect registration of the coating in relation to the embossing, and provides the possibility of better penetration of the coating into the substrate. In this method, an external embossing sleeve having a pattern is outside the screen and internal back-up roll of a rotary screen coating device. Such a configuration provides embossing and coating on the raised portion of the bosses produced by the sleeve because of through holes in the sleeve that permit the coating material to be deposited on the substrate. In other words, coating only occurs on the areas of the substrate that have not been depressed by the embossing sleeve and are higher than the valleys. The solid backup roll can be made of metal.

Additionally, it has been found to be advantageous to have an uncoated area, e.g. open area, of at least about 40%.

Alternately, the raised portion and the valley of the embossment can both be coated, with the same or different coating.

When liquid is introduced into an absorbent core having polymer coating on boss areas, the liquid can quickly absorb into the structure through uncoated valley portion. Without being bound by any particular theory, because the valley portion has been compressed, its high capillary force allows fluid to spread quickly while the low density portion underneath the polymer coating provide absorbency. The fluid is held under the coating level providing a structure that is dry to the touch.

Alternately, a coating can be deposited on the floor area, at least one of the sidewalls of the embossed area, or both. Such methods are disclosed in co-pending European application entitled ENHANCED EMBOSSING AND RELATED METHODS, filed concurrently herewith, claiming priority from USSN 10/609745 (Attorney's ref: P038154EP).

### Garment Facing Layer

The garment facing layer of the present invention may be pliant and is typically referred to as a backsheet or barrier layer. The exterior of the garment facing layer forms the garment-facing surface of the absorbent article and, typically, is impermeable to fluids.

In one embodiment, the garment facing layer may be any thin, flexible, fluid impermeable material, such as a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene.

In one embodiment, the garment facing layer may be any polymer, such as, PCL, PLA, and PVOH.

Additionally, the garment facing layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

The thickness of the backsheet when formed from a polymeric film typically is about 0.0005 inch (0.0125 mm) to about 0.002 inch (0.051 mm).

In an embodiment of the present invention where the absorbent article is flushable, the garment facing layer is made from paper and may include additives, such as, polyvinyl alcohol (PVOH) and carboxymethylcellulose (CMC). Such paper may be formed by coating a water-soluble PVOH resin to paper (cellulose fibers) on one side to form a laminated structure. The paper itself is not water soluble but can be biodegraded. PVOH dissolves upon exposure to fluid, e.g., water.

The material used for the garment facing layer may be adhered to the absorbent core by any method known to one of skill in the art. Such methods of adherence include mechanical methods, such as, heating, embossing, crimping, hooks, and the like, and chemical methods, such as, adhesives, including latex, solvents, and the like. Where an adhesive is used, one useful adhesive is a hot melt adhesive that is water soluble, but is stable when body exudates contact it. An example of such an adhesive is a sulfopolyester adhesive marketed as Cycloflex® brand from National Starch.

In one embodiment, the garment facing layer can be extruded or coated directly onto the substrate.

In one embodiment, an absorbent core can be made of an absorbent core made of a wet laid pulp, available as Valucore brand from Rayonier, which is laminated using hot melt adhesive to a 1 mil layer of PCL (available from Union Carbide/Dow, Midland, MI).

The absorbent structure may also be formed from an absorbent core and a film, which has been laminated together to form a unitary structure. The film may be any water insoluble, biodegradable polymers, such as, PCL and PVOH.

Any material for the garment facing layer can be laminated to the absorbent core by any method known in the art, including, but not limited to, sonic welding, ionic bonding, adhesives, heating and the like.

### Garment Attachment

Secure attachment of absorbent article of the claimed invention to the garment contributes to maintaining the feeling of the user that the absorbent article and the garment are one in the same, i.e., permits the absorbent article to move with the underwear.

The absorbent article of the present invention may be applied to the crotch by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may include pressure sensitive adhesive that is applied as dots, strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to polystyrene, polyalpha-methylstyrene, polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton® elastomers from Kraton Ploymer Co., Vector® elastomers from Dexco, Solprene® from Enichem Elastomers and Stereon® from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate, vinyl silane or maleic anhydride. Commercial examples of these types of polymers include Ateva® (polymers from AT plastics), Nucrel® ( polymers from DuPont), Escor® (from Exxon Chemical).

Where adhesive is used, a release strip may be applied to protect the adhesive on the absorbent article prior to attaching the absorbent article to the crotch. The release strip can be formed from any suitable sheet-like material adheres with sufficient tenacity to the adhesive to remain in place prior to use but which can be readily removed when the absorbent article is to be used. Optionally, a coating may be applied to release strip to improve the ease of removabilty of the release strip from the adhesive. Any coating capable of achieving this result may be used, e.g., silicone.

The above discussion is provided to illustrate the present invention, as well as certain physical properties and uses thereof. Such discussion is illustrative only and is not intended to limit the scope of the invention in any way.

## Claims

1. An absorbent article comprising:
an absorbent structure, the absorbent structure comprising an absorbent core and a plurality of discrete entities deposited on the absorbent core, wherein the combination of absorbent core and discrete entities form a body facing surface.

2. An absorbent article of claim 1, further comprising a garment facing layer juxtaposed to the absorbent structure opposite the body facing surface.

3. An absorbent article of claim 2, wherein the garment facing layer is a polymeric film, a treated fluid-pervious material, a water-insoluble, biodegradable polymer or a mixture thereof.

4. An absorbent article of claim 1, wherein the absorbent core is laminated to a water-insoluble, biodegradable polymer to form a garment facing surface opposite the body facing surface.

5. An absorbent article of any one of claims 1 to 4, wherein the discrete entities comprise hot melt adhesive.

6. An absorbent article of any one of claims 1 to 5, wherein the discrete entities are dots.

7. An absorbent article of any one of claims 1 to 6, wherein the absorbent core comprises cellulose fibers, cotton fibers, rayon fibers, superabsorbent fibers, superabsorbent particles; sphagnum; absorbent foam or a mixture thereof.

8. An absorbent article of claim 7, wherein the absorbent core comprises at least about 1% SAP.

9. An absorbent article of any one of claims 1 to 8, wherein the discrete entities comprise up to about 60% of the body facing surface.

10. An absorbent article of any one of claims 1 to 9, wherein the discrete entities are printed onto the absorbent core.

11. An absorbent article of any one of claims 1 to 10, wherein the absorbent core is embossed to form raised areas and valley areas.

12. An absorbent article of claim 11, wherein the discrete entities are deposited on the raised areas of the absorbent core.

13. An absorbent article of claim 11 or claim 12, wherein at least one valley areas has a coating material deposited therein.

14. An absorbent article of any one of claims 11 to 13, wherein embossing and depositing are carried out at a single station.
